# EUROPÄISCHE PATENTANMELDUNG

(11) **EP 2 277 545 A1**
(43) Veröffentlichungstag der Anmeldung: **26.01.2011**
(21) Anmeldenummer: 10163367.5
(22) Anmeldetag: 20.05.2010
(51) Int. Cl.: A61K 41/00, A61K 9/00, A61K 47/48

(54) **Partikel mit induzierbarer Formänderung**

(30) Priorität: 24.06.2009 DE 102009027151
(71) Anmelder: GKSS-Forschungszentrum Geesthacht GmbH, 21502 Geesthacht (DE)
(72) Erfinder: Lendlein, Prof. Dr., 14167, Berlin (DE); Wischke, Dr. Christian, 14513, Teltow (DE); Neffe, Dr. Axel Thomas, 10965, Berlin (DE)
(74) Vertreter: Gulde Hengelhaupt Ziebig & Schneider

(57) **Zusammenfassung**

Die Erfindung betrifft Partikel, die eine stimulierbare Formänderung aufzeigen und Aufnahme in aktive Zellen steuerbar ist. Die Partikel können als Trägersysteme für bioaktive Moleküle oder Diagnostika dienen und besitzen in ihrer sphärischen Form eine Größe, die die Aufnahme in eine Zelle erlaubt. Jedes Partikel nimmt eine temporäre, nicht-sphärische und damit nicht oder nur geringfügig aufnehmbare Form ein, die durch einen geeigneten Stimulus in eine sphärische und damit aufnehmbare Form überführbar ist.

## Beschreibung

Die Erfindung betrifft Partikel, die eine induzierbare Formänderung aufzeigen und deren Aufnahme in Zellen aktiv steuerbar ist. Diese Partikel können als Trägersysteme für bioaktive Moleküle oder Diagnostika dienen.

### Stand der Technik

In vielen medizinischen Anwendungsbereichen besteht anhaltender Bedarf, die Freisetzung von Wirkstoffen zeitlich und lokal zu steuern, zum Beispiel im Rahmen einer medikamentösen Therapie bei Mensch und Tier. Weiterhin besteht Bedarf, Diagnostika bei Mensch und Tier an definierten Orten oder in definierten Geweben des Organismus lokal zu konzentrieren zum Beispiel für bildgebende Verfahren. Von besonderer Bedeutung für Physiologie und Pathologie sind vielfach biologische Prozesse, die unter Beteiligung des Immunsystems ablaufen.

Für eine immunologische Prozessierung von beispielsweise Fremdteilchen wie Bakterien oder Partikeln muss zunächst eine Aufnahme der Fremdteilchen in aktive Zellen, insbesondere antigenpräsentierende Zellen erfolgen. Für therapeutische oder diagnostische Anwendungsfelder ist es nun wünschenswert, den Zeitpunkt der zellulären Aufnahme von Teilchen steuern zu können. Es besteht daher ein Bedarf nach Lösungen, die eine schaltbare biologische Erkennung und zelluläre Aufnahme von Teilchen erlauben.

Bekannt ist, dass formstabile Partikel von phagozytotisch-aktiven Zellen in Abhängigkeit von der Partikelform entweder aufgenommen werden können oder nicht bzw. stark vermindert aufgenommen werden (Proc. Natl. Acad. Sci. 103 (2006) S. 4930 - 4934; Pharm. Res. 25 (2008) S. 1815 - 1821). So haben Untersuchungen zum Einfluss der Partikelgeometrie auf die Erkennung durch aktive Zellen ergeben, dass eine verminderte Aufnahmerate bei bestimmten Geometrien, zum Beispiel wurmartigen Partikeln, verglichen mit sphärischen Partikeln besteht.

### Zusammenfassung der Erfindung

Mit Hilfe der erfindungsgemäßen Partikeln werden ein oder mehrere der angesprochenen Probleme gelöst oder zumindest gemindert. Es werden Partikel bereitgestellt, die die Befähigung zur induzierbaren Formänderung besitzen. Ein zur zellulären Aufnahme geeignetes Partikel nimmt vor der Anwendung eine temporäre, nicht-sphärische und damit nicht oder nur geringfügig aufnehmbare Form ein, die durch einen geeigneten Stimulus in eine sphärische und damit in Zellen aufnehmbare Form überführbar ist. Jedes Partikel nimmt demnach eine temporäre, nicht-sphärische und damit nicht oder nur geringfügig durch aktive Zellen aufnehmbare Form ein, die durch einen geeigneten Stimulus in eine sphärische und damit aufnehmbare Form überführbar ist.

Der Erfindung liegt die Erkenntnis zugrunde, Partikel zunächst in einer für die zelluläre Aufnahme ungünstigen temporär-programmierten Form bereitzustellen. Induziert durch einen nach der Applikation der Partikel wirkenden Auslöser nehmen die Partikel eine sphärische Kontur an, die eine gegenüber der Ausgangsform verstärkte zelluläre Aufnahme ermöglicht. Mit anderen Worten, erst nach Rückerlangen der permanenten sphärischen Form wird das Partikel vermehrt durch die aktiven Zellen aufgenommen. Im Falle von Partikeln, die bioaktive Moleküle erhalten, können sich dann im Inneren der aktiven Zellen die gewünschten, durch den oder die bioaktiven Moleküle bedingten Wirkungen entfalten. Derartige technische Lösungen, die durch eine gezielte Änderung der Partikelform eine schaltbare biologische Erkennung der Partikel für die zelluläre Aufnahme ermöglichen, sind bisher unbekannt. Ein Teil des Partikels oder das gesamte Partikel besteht demnach - neben dem gegebenenfalls einzubringenden Wirkstoff und optional weiteren Hilfsstoffen - aus einem Material, das in einer temporären Form fixiert ist und in dieser zur Anwendung gebracht werden kann. Unter Anwendung eines geeigneten Stimulus erfolgt die Rückstellung des Materials und damit des Gesamtpartikels in die programmierte sphärische Form, d.h. von der temporären, nichtaufnehmbaren Form in seine durch aktive Zellen aufnehmbare Form.

Der Begriff "Partikel" im Sinne der Anmeldung bezieht sich auf Teilchen unterschiedlichster struktureller Ausprägung. Das Partikel, vollständig oder in Teilen, kann insbesondere eine kompakte zusammenhängende Matrix besitzen, eine poröse Struktur aufweisen, ein mit Flüssigkeiten gefülltes Netzwerk oder ein interpenetrierendes Netzwerk sein, einen kapselartigen Aufbau mit Kern und ein oder mehreren Hüllschichten zeigen, oder aus einer Vielzahl von Einzel-Teilchen zusammengesetzt sein. Für die Zwecke der Erfindung von Bedeutung ist, dass das Partikel zu einer gerichteten Formänderung ausgehend von einer nicht-sphärischen hin zu einer sphärischen Form befähigt ist, die durch einen geeigneten Stimulus induziert wird. Vorzugsweise besteht das Partikel aus einem porösem Material / Scaffold oder aus einer Vielzahl kleinerer Teilchen.

Der Begriff "Stimulus" im Sinne der Anmeldung ist als jedwedes Einwirken auf das Partikel zu verstehen, das eine Rückstellung des Partikels in die permanente sphärische Form bewirkt. Auslöser kann beispielsweise eine Temperaturänderung oder Änderung der chemischen Umgebung, wie pH-Wert oder dergleichen, sein. Denkbar ist auch, dass die Rückstellung durch Abbau von Strukturen des Partikels ausgelöst wird, die das Partikel in der nicht sphärischen Form fixieren.

Der Begriff "Aufnahme" beschreibt die Inkorporierung von Partikeln in aktive Zellen, insbesondere in Zellen mit Befähigung zur Antigenpräsentation wie Makrophagen, Dendritische Zellen, Langerhans-Zellen, B-Lymphozyten, etc. Die Aufnahme kann durch alle biologisch möglichen Mechanismen inklusive Rezeptor-vermittelter und nicht-Rezeptor-vermittelter Mechanismen erfolgen. In einer Ausführungsform erfolgt die zelluläre Aufnahme durch Phagozytose, d.h. das Partikel ist vorzugsweise zur Aufnahme durch Phagocytose in Zellen hergerichtet.

Der Begriff "Formgedächtnismaterial", wie er vorliegend verwendet wird, bezieht sich auf ein Material, das von der vorgegebenen permanenten Gestalt durch geeignete Formgebungsverfahren in eine temporäre Form überführt und in dieser fixiert werden kann. Beim Anlegen eines externen Stimulus geht das Material wieder in die ursprüngliche, permanente Form über. Die Deformation und Fixierung der temporären Form wird als Programmierung bezeichnet. Der Übergang von der temporären in die permanente Gestalt wird unter dem Begriff Rückstellung zusammengefasst. In diesem Sinne ist auch der hier verwendete Begriff "Formgedächtniseffekt" zu verstehen, der sich jedoch nicht nur auf die Verwendung von Formgedächtnismaterialien beschränkt, sondern allgemein eine durch einen Stimulus induzierte Formänderung beschreibt.

Das Partikel mit induzierbarer Formänderung besteht aus Materialien, die biostabil oder vollständig oder partiell biodegradierbar sein können. In einer bevorzugten Ausführungsform besteht das Partikel ganz oder in Teilen aus einem Formgedächtnismaterial, insbesondere ein Formgedächtnispolymer, Formgedächtnishydrogel, oder Stimuli-sensitives Gel. Das Formgedächtnismaterial ist vorzugsweise ein Formgedächtnispolymer mit Schaltsegmenten und Netzpunkten, dass oft durch einen geringen Wassergehalt < 5% (m/m) bzw. eine geringe Kapazität zur Wasseraufnahme < 5% (m/m) gekennzeichnet ist. In einer weiteren bevorzugten Ausführungsform besteht das Partikel ganz oder in Teilen aus einem Formgedächtnisgel mit einem hohen Wasssergehalt > 5%. In einer weiteren bevorzugten Ausführungsform besteht das Partikel ganz oder in Teilen aus einem Stimuli-sensitiven Gel. In einer weiteren bevorzugten Ausführungsform ist das Partikel eine Kapsel mit einem Kern und einer Hülle die wiederum aus mehreren Schichten aufgebaut sein kann.

In einer bevorzugten Variante besteht der Kapselkern aus einem quellbaren Gel, einem Stimuli-sensitiven Gel, oder einem Formgedächtnis-Gel. Die Kapsel hat eine permanente sphärische Form, die in die temporäre, nicht-sphärische Form überführt werden kann. Die Überführung der Kapsel in die nicht-sphärische Form und die Fixierung in dieser Form erfolgt dabei unter Zuhilfenahme der Kapselhülle. In einer bevorzugten Variante besteht die Kapselhülle vollständig oder zu Teilen aus einem Formgedächtnispolymer. In einer weiteren bevorzugten Variante besteht die Kapselhülle aus einer wachsartigen Substanz. Hierbei wird der Kern, der eine sphärische Grundform besitzt, durch die Formgebung der Kapselhülle elastisch deformiert in die temporäre, nicht-sphärische und damit nicht oder nur geringfügig aufnehmbare Form gezwungen.

Die Partikel in ihrer sphärischen Form haben vorzugsweise eine phagozytierbare Größe. Ein mittlerer Partikeldurchmesser der Partikel in der aufnehmbaren Form beträgt vorzugsweise 0,1 bis 100 µm, insbesondere 0,5 bis 10 µm. Die zur Anwendung gebrachten Partikel besitzen eine nicht- oder nur begrenzt aufnehmbare Form, vorzugsweise liegt der Partikel in dieser temporären Form als oblates Ellipsoid, prolates Ellipsoid, elliptische Scheibe, rechteckige Scheibe, in sogenannter UFO-Form (vergl. Proc. Natl. Acad. Sci. 103 (2006) S. 4930 - 4934), stabförmig oder wurmförmig vor. Durch einen geeigneten Stimulus wird die temporäre Form in eine sphärische, aufnehmbare Form überführt.

Das Partikel kann als Träger für biologisch aktive Substanzen dienen. Dabei umfasst der Begriff "biologisch aktive Substanz" bzw. "Wirkstoff" alle pharmakologisch aktiven Substanzen inklusive immunologisches Material oder Diagnostika sowie Mischungen derselben. Der Ausdruck umfasst auch Arzneimittel, Kontrastmittel oder radioaktive Labels und dergleichen. Er umfasst auch Vorstufen, die im Körper zu ihrer aktiven Form umgewandelt werden, aktive und inaktive Konformationen und Isomere, und pharmakologisch und/oder immunologisch aktive Fragmente derselben. Insbesondere ist der Wirkstoff ein Protein, Peptid, Glykoprotein, Glykopeptid, Glykolipid, Lipoprotein, Lipopeptid, Cytokin, Polysaccharid, DNA, RNA oder Agonist oder Antagonist bzw. Inhibitor von Rezeptoren, Enzymen oder Transportern der zur Partikelaufnahme befähigten aktiven Zellen. Weiterhin fallen unter den Begriff auch Impfstoffe aller Art inklusive Bakterien, Viren, ihrer Spaltprodukte und synthetischen Analoga, sowie Immunomodulatoren und Immunopotentiatoren. Weiterhin fallen unter den Begriff Cytostatika, Immunosupressiva, Glucocorticoide, Toll-like Rezeptor-Liganden, NOD-like Rezeptor-Liganden, Antiproliferativa, Enzyminhibitoren, Statine. Weiterhin fallen unter den Begriff hydrophile, hydrophobe, und amphiphile Substanzen.

Wirkstoffbeladene Partikel mit induzierbarer Formänderung können dazu dienen, die durch diese Wirkstoffe hervorgerufenen biologischen Effekte zeitlich und lokal schaltbar werden. Der Wirkstoff ist dabei vorzugsweise in die Partikelmatrix eingebettet oder der Partikel weist eine Kavität auf, in die der Wirkstoff eingebracht ist. Die Einbettung des Wirkstoffs kann auch durch eine kovalente Anbindung an das Matrixmaterial erfolgen, beispielsweise durch eine intrazellulär hydrolytisch, enzymatisch oder redox-chemisch spaltbare Bindung.

Die Verwendung von Impfstoffen im Zusammenspiel mit den erfindungsgemäßen Partikeln eröffnet die Möglichkeit, anstelle einer bekannten Mehrfachimpfung eine Mischung aus sofort verfügbarem Impfstoff mit dem zunächst nicht-phagozytierbaren und damit nicht immunologisch wirkenden Partikeln zu applizieren. Diese werden erst später durch gezielt induzierte Formveränderung zur immunologischen Erkennung, Phagozytose und intrazellulären immunologischen Prozessierung freigegeben.

Vorzugsweise ist eine Freisetzungsrate des Wirkstoffs aus den Partikeln in der temporären Form geringer als in der sphärischen Form. Mit anderen Worten, induziert mit der Formänderung wird beispielsweise die Zugänglichkeit zu Kavitäten vereinfacht, so dass eine Diffusion des Wirkstoffs forciert wird.

Die erfindungsgemäß eingesetzten Partikel bestehen demnach unter anderem neben dem gegebenenfalls vorhandenen Wirkstoff (i) aus Formgedächtnispolymeren, (ii) aus Formgedächtnisgelen, (iii) aus stimuli-sensitiven Hydrogelen oder (iv) sind ein oder mehrschichtige Kapseln mit einem Kern einer definierten Form, der durch eine oder mehrere Schichten der Kapselhülle aus einem anderen Material in der programmierten Form gehalten wird.

Die Partikel können entweder direkt während der Formung der permanenten Partikelform mit allen bekannten Verfahren zur Partikelherstellung (z.B. Emulsions-, Sprüh-, Koazerationsprozesse; Int. J. Pharm. 364 (2008) 298-327) oder in nachgeschalteten Arbeitsschritten mit bioaktiven Molekülen (z.B. durch Quellen in geeigneten Lösungen der Substanzen) beladen werden. Die Substanzen können diffusionsgesteuert oder degradationsgesteuert freigesetzt werden. In verschiedenen Ausführungsformen kann die Wirkstofffreisetzung sowohl unabhängig von der geschalteten Formveränderung, zeitgleich, oder durch diese ausgelöst initiiert werden. Die Freisetzung der Partikelbeladung erfolgt im Zeitraum von Sekunden bis Jahren, bevorzugt über 1 Stunde bis 4 Wochen.

Die zur Formgebung einer temporären Form des Partikels verwendeten Materialien können sowohl niedermolekulare Substanzen als auch Polymere sein, bevorzugt werden Formgedächtnispolymere, Formgedächtnishydrogele, polymerbasierte stimuli-sensitive Hydrogele, sowie im Falle der Kapselhüllen wachsartige Substanzen verwendet. Diese Materialien sind biokompatibel bezogen auf die Anwendung bei Mensch und Tier sowie diagnostische Anwendungen in vivo, ex vivo und in vitro. Die Partikel bestehen aus oben genannten Materialien, gegebenenfalls Hilfsstoffen < 5% (m/m) und Wirkstoff (0,001% - 80% [m/m]) sowie gegebenenfalls einer oder mehrerer Schichten eines Beschichtungsmaterials. Sie können i) biostabil sein, d.h. der Partikelkern wird in biologischem Milieu über mehrere Jahre nicht abgebaut, ii) vollständig biodegradierbar sein zu wasserlöslichen und exkretierbaren Mono- bis Oligomeren, iii) partiell biodegradierbar sein, d.h. es erfolgt ein Abbau der gesamten Partikelmatrix zu aus dem Körper ausscheidbaren und/oder nicht ausscheidbaren Bruchstücken, oder iv) ausscheidbar sein auf anderen Wegen als durch chemischen oder biochemischen Abbau, zum Beispiel Zerfall des Partikels aus physikalischen Aggregaten in Einzelteilchen oder Moleküle von ausscheidbarer Größe. Der Begriff "Bioabbau" umfasst dabei unter anderem enzymatische Mechanismen, hydrolytische Mechanismen, und Redox-Reaktionen wie beispielsweise die Spaltung von Disulfid-Brücken.

Als Formgedächtnismaterial können Stimuli-sensitive Formgedächtnsipolymere und Formgedächtnisgele mit Formgedächtniseigenschaften verwendet werden, deren Formveränderung zum Beispiel durch Wärmeübertragung (direkten Kontakt oder indirekt durch elektrische, magnetische oder sonstige Stimuli), unter Einwirkung von Lösungsmitteln und Weichmachern einschließlich Wasser, Licht oder chemische bzw. biochemische Stimuli ausgelöst wird. Es kann sich um physikalisch oder kovalent vernetzte Polymernetzwerke handeln.

Als Partikelmatrix können ebenfalls Stimuli-sensitive Gele verwendet werden. Diese besitzen einen hohen Gehalt von Lösungsmitteln >5% (m/m), bevorzugt im Bereich von 20-99,999% (m/m). Hierbei handelt es sich um ein biokompatibles Lösungsmittel bezogen auf das Anwendungsfeld wie z.B. Dimethylsulfoxid, N-Methylpyrrolidon, bevorzugt jedoch um Wasser (das Material wird dann als Hydrogel bezeichnet). Als Gelbildner können sowohl kleine Moleküle (supramolekulare Gele, z.B. Chem. Soc. Rev., 2008, 37, 2699-2715) als auch Polymernetzwerke genutzt werden. Sie können entsprechend dem derzeitigen Stand der Wissenschaft unter anderen in Reaktion auf Änderungen des pH-Wertes, der Temperatur, lonenstärke, elektrische oder magnetischer Felder, sowie enzymgesteuert ihre Dimension verändern.

Neben einer Fixierung der Partikel in einer nicht-sphärischen Form und schaltbaren Überführung in eine sphärische Form mit Hilfe des Formgedächtnispolymers, Formgedächtnisgels, Stimuli-sensitiven Gels können auch Kapseln mit einem Partikelkern aus quellbaren Gelen, Formgedächtnisgelen, oder Stimulis-sensitiven Gelen genutzt werden. Dabei besitzt der Partikel-Kern eine vorgegebene sphärische Form, die ohne äußeren Zwang eingenommen wird. Die Überführung in eine nicht aufnehmbare, d. h. eine nicht-sphärische Partikelform erfolgt für diese Partikel mithilfe einer Beschichtung aus einem anderen Material. Dieses andere Material kann ein Formgedächtnismaterial sein. Alternativ kann das andere Material ein Material ohne Formgedächtniseffekt, zum Beispiel eine wachsartige Substanz darstellen. Diese Beschichtung wird vor der Überführung in eine nicht-sphärische Partikelform aufgebracht. Die Beschichtung ist hierbei geeignet, den Rückstellkräften des Partikelkerns zu widerstehen und damit den Erhalt der dann programmierten Form sicherzustellen. Durch Einwirkung geeigneter Stimuli, zum Beispiel über thermische, elektromagnetische, chemische, oder biochemische Stimuli, allmählichen Abbau im Körper oder thermischen Übergänge, wie Schmelzen bei Temperaturanstieg, erfolgt ein Verlust der mechanischen Stabilität der Beschichtung. Wenn die Beschichtung durch Anwendung von Stimuli in die sphärische Form überführt wird oder bei Abbau der Beschichtung die Rückstellkräfte des PartikelKerns überwiegen, erfolgt die Formrückstellung des Partikels zur ursprünglichen Form, d.h. zur sphärischen Form.

Sofern für die Herstellung Kapselhülle ein Formgedächtnismaterial verwendet wird, braucht der Kern lediglich aus einem deformierbaren Material zu bestehen. Durch Einwirkung geeigneter Stimuli auf die Kapselhülle, zum Beispiel über thermische, elektromagnetische, chemische, oder biochemische Stimuli, wird dann die Rückstellung initiiert.

Die Programmierung der Partikel erfolgt an Einzelpartikeln oder aber in einem zweckmäßigen Aufbau durch zeitgleiche Programmierung zahlreicher Partikel, zum Beispiel zwischen Platten mit gegebenenfalls spezieller Oberflächenbeschaffenheit und Struktur oder zum Beispiel durch eine Methode unter Nutzung von Filmen. Bei der Filmmethode erfolgt die Programmierung der kugelförmigen zu nicht-sphärischen Partikeln, indem sie in eine Polymermatrix wie einen Polymerfilm, zum Beispiel einen wasserlöslichen Polymerfilm eingearbeitet werden, dieser bei geeigneten Bedingungen (zum Beispiel erhöhter Temperatur) in ein, zwei, oder drei Dimensionen verstreckt oder fixiert wird, und anschließend die nicht-kugelförmigen Partikel durch Auflösen des Films isoliert werden. Genannter Prozess ist in der Literatur zur irreversiblen Deformation von Polystyrol-Partikeln beschrieben worden (Colloid Polym. Sci. 271 (1993) S. 469-479; Proc. Natl. Acad. Sci. 104 (2007) S. 11901-11904).

Partikel mit Durchmessern kleiner als 10 µm, insbesondere kleiner 5 µm, können bei Applikation ins Gewebe (z.B. intramuskulär, subkutan oder intradermal) durch Makrophagen oder dendritische Zellen aufgenommen und über die lymphatischen Organe abtransportiert werden. Spezielle Zellen, so genannte foreign body giant cells, können auch größere Partikel aufnehmen. In vitro konnte gezeigt werden, dass Makrophagen nicht-kugelförmige Mikropartikel in reduziertem Maße phagozytieren. Die erfindungsgemäßen nichtsphärischen Partikel mit reduzierter zellulären Aufnahme können nach Injektion durch eine von außen stimulierte Formveränderung in eine Kugelform überführt, in aktive Zellen aufgenommen, im Zellinneren prozessiert, gegebenenfalls in die lymphatischen Organe transportiert, und eine gegebenenfalls vorhanden Beladung immunologisch erkannt werden. Dies kann beispielsweise für ein neues Prinzip von Mehrfachimpfungen bei Mensch und Tier mit nur einmaliger Applikation genutzt werden, wobei Mischungen von sofort verfügbarem Impfstoff (gelöst oder in kugelförmigen Partikeln) und nicht kugelförmigen Partikeln verwendet werden, bei denen anstelle einer weiteren Applikation Partikel fraktioniert in eine immunologisch erkennbare Form überführt werden. Weiterhin kann dieses Prinzip für diagnostische Fragestellungen, z.B. eine Aufklärung von physiologisch, pathologisch oder durch Immunoseneszenz bedingten Veränderungen der phagozytotischen Aktivität von Zellen genutzt werden.

Die Erfindung wird nachfolgend in einem Ausführungsbeispiel und anhand von Zeichnungen näher erläutert. Es zeigen:
- Fig. 1: eine schematische Illustration zweier Wege der Ausführbarkeit der erfindungsgemäßen Partikel mit Formgedächtniseffekt;
- Fig. 2: Aspektverhältnisse von Mikropartikeln in programmierter Form und nach Formrückstellung; und
- Fig. 3: mikroskopische Aufnahmen von Mikropartikeln aus einem Formgedächtnispolymer in temporärer, programmierter Form und nach Formrückstellung zur permanenten Form.

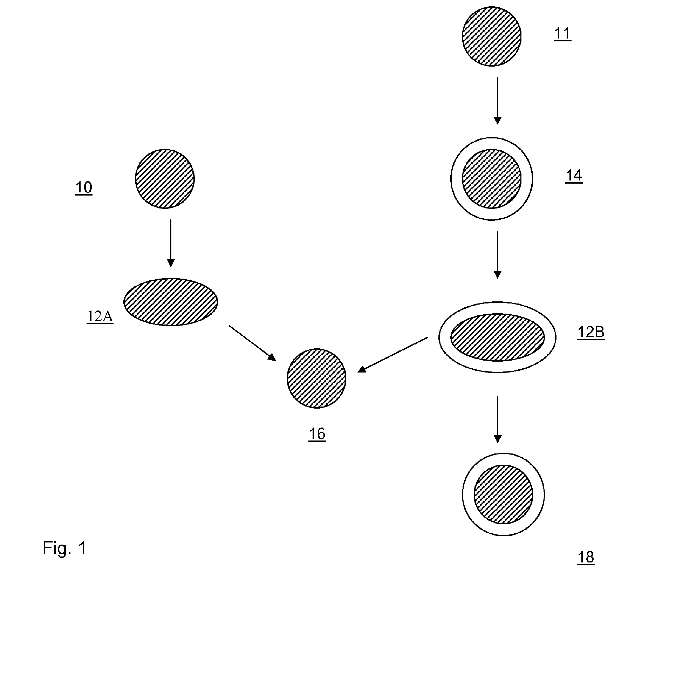

In einem ersten Schritt werden sphärische Partikel 10 mit einer zur zellulären Aufnahme befähigten Formgebung aus einem für die gewünschte Applikation geeigneten Material hergestellt. In der auf der linken Seite der Figur 1 dargestellten Ausführungsform ist das Material ein Formgedächtnispolymer, ein Formgedächtnisgel oder ein Stimuli-sensitives Gel, das gegebenenfalls mit einem Wirkstoff beladen ist. Durch Programmierung wird das Partikel 10 in einer temporären, nicht-sphärischen und damit nicht oder nur geringfügig phagozytierbare Form 12A fixiert. Durch einen geeigneten Stimulus, zum Beispiel eine Änderung von Temperatur, pH-Wert, lonenstärke oder Einwirkung von Licht, wird das Partikel in die sphärische und damit phagozytierbare Form 16 überführt und kann nach Phagozytose die gegebenenfalls gewünschten biologischen Effekte bewirken.

Nach einer zweiten Variante, die auf der rechten Seite der Figur 1 illustriert ist, besteht das sphärische Partikel 11 aus einem quellbaren Gel, einem Formgedächtnisgel oder einem Stimuli-sensitiven Gel und ist elastisch oder plastisch verformbar. Das Partikel 11 wird nach dieser Variante zunächst mit einem geeigneten Material, zum Beispiel einem Wachs oder einem Formgedächtnispolymer, beschichtet (beschichtetes Partikel 14) und durch ein Formgebungsverfahren dann in einer temporären, nicht-sphärischen und damit nicht oder nur geringfügig phagozytierbare Form 12B fixiert. Durch einen geeigneten Stimulus, hier zum Beispiel hydrolytischen Zerfall der Beschichtung, wird das Partikel 12B in die sphärische und damit phagozytierbare Form 16 überführt. Durch andere geeignete Stimuli ohne Zerfall der Beschichtung erfolgt der Übergang des Partikels in die sphärische und damit phagozytierbare Form 18, die der Form 14 gleichen kann.

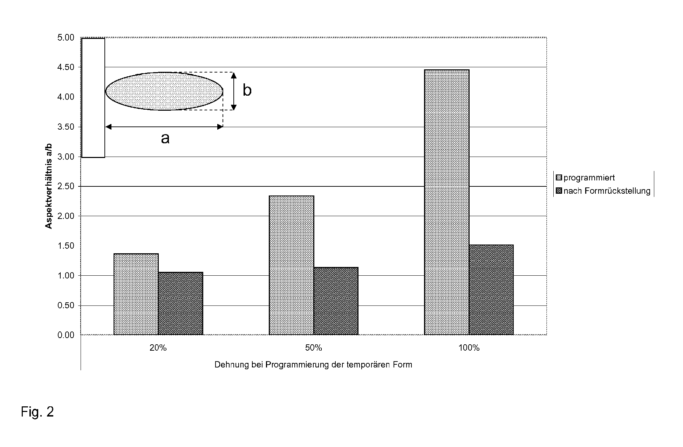

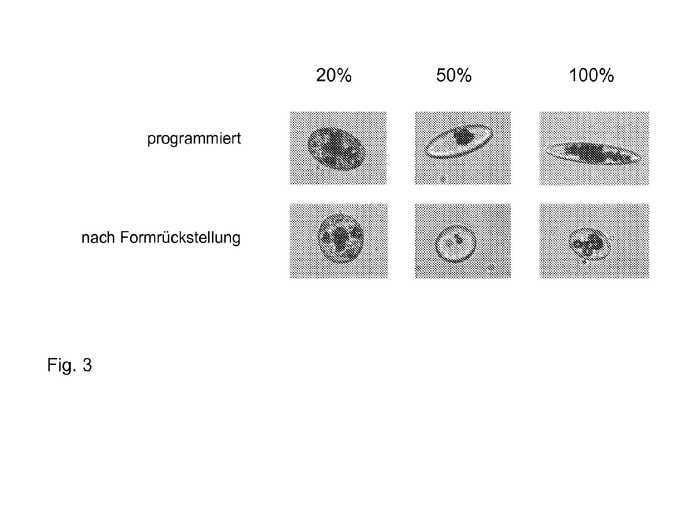

Fig. 2 zeigt Aspektverhältnisse von Mikropartikeln in programmierter Form und nach Formrückstellung. Fig. 3 sind mikroskopische Aufnahmen der zugehörigen Mikropartikel aus einem Formgedächtnispolymer zu entnehmen. Die Partikel liegen in temporärer, programmierter Form vor mit Dehnungen von 20%, 50% beziehungsweise 100%. Nach Formrückstellung zur permanenten Form liegen wieder sphärische Partikel vor.

## Patentansprüche

1. Partikel mit induzierbarer Formänderung, wobei ein zur zellulären Aufnahme geeignetes Partikel vor der Anwendung eine temporäre, nicht-sphärische und damit nicht oder nur geringfügig aufnehmbare Form einnimmt, die durch einen geeigneten Stimulus in eine sphärische und damit in Zellen aufnehmbare Form überführbar ist.

2. Partikel nach Anspruch 1, bei dem das Partikel ganz oder in Teilen aus einem Formgedächtnispolymer, Formgedächtnishydrogel, oder Stimuli-sensitivem Gel besteht.

3. Partikel nach Anspruch 1, bei dem das Partikel eine Kapsel ist mit einem deformierbaren Kern aus einem quellbaren Gel, einem Formgedächtnisgel, oder einem Stimuli-sensitiven Gel und einer den Kern umhüllenden Kapselhülle aus dem Formgedächtnispolymer.

4. Partikel nach einem der vorhergehenden Ansprüche, das aus einem porösem Material / Scaffold oder aus einer Vielzahl kleinerer Teilchen besteht.

5. Partikel nach Anspruch 1, bei dem das Partikel eine Kapsel ist mit einem deformierbaren Kern aus einem quellbaren Gel, einem Formgedächtnisgel, oder einem Stimuli-sensitiven Gel und einer den Kern umhüllenden Kapselhülle aus einem nicht Formgedächtnismaterial, wobei der Kern eine sphärische Grundform besitzt, die jedoch durch die Formgebung der Kapselhülle elastisch deformiert in die temporäre, nicht-sphärische und damit nicht oder nur geringfügig in Zellen aufnehmbare Form gezwungen ist.

6. Partikel nach einem der vorhergehenden Ansprüche, wobei ein mittlerer Partikeldurchmesser des Partikels in der in Zellen aufnehmbaren Form 0,1 bis 100 µm beträgt.

7. Partikel nach Anspruch 6, wobei der mittlere Partikeldurchmesser des Partikels in der in Zellen aufnehmbaren Form 0,5 bis 10 µm beträgt.

8. Partikel nach einem der vorhergehenden Ansprüche, wobei das Partikel in der temporären Form als oblates Ellipsoid, prolates Ellipsoid, elliptische Scheibe, rechteckige Scheibe, in UFO-Form, stabförmiges Partikel oder wurmförmiges Partikel vorliegt.

9. Partikel nach einem der vorhergehenden Ansprüche, bei dem das Partikel einen oder mehrere Wirkstoffe enthält.

10. Partikel nach Anspruch 9, wobei der Wirkstoff in die Partikelmatrix oder Teile der Partikelmatrix eingebettet oder gebunden ist.

11. Partikel nach Anspruch 9 oder 10, wobei eine Freisetzungsrate des Wirkstoffs aus dem Partikel in der temporären Form geringer ist als in der phagozytierbaren Form.

12. Partikel nach einem der Ansprüche 9 bis 11, wobei der Wirkstoff ein Arzneistoff, ein Impfstoff, Proteine, Peptide, Glykoproteine, Glykopeptide, Lipoproteine, Lipopeptide, Glykolipide, Cytokine, Polysaccharide, DNA, RNA, Glukokorticoide, Immunsuppressiva, Immunmodulatoren, Immunpotentiatoren, Cytostatika, Toll-like Rezeptor-Liganden, NOD-like Rezeptor-Liganden, Antiproliferativa, Enzyminhibitoren, Statine oder Tenside gehört.

13. Partikel nach den vorhergehenden Ansprüchen, bei dem das Partikel zur Aufnahme durch Phagocytose in Zellen hergerichtet ist.
